# EUROPÄISCHE PATENTSCHRIFT

(11) **EP 2 026 760 B1**
(45) Veröffentlichungstag und Bekanntmachung des Hinweises auf die Patenterteilung: **03.12.2014**
(21) Anmeldenummer: 07724066.1
(22) Anmeldetag: 05.04.2007
(51) Int. Cl.: A61K 9/00, A61K 9/70

(54) **TRANSDERMALES THERAPEUTISCHES SYSTEM**
TRANSDERMAL THERAPEUTIC SYSTEM
SYSTÈME THÉRAPEUTIQUE PERCUTANÉ

(30) Priorität: 17.05.2006 DE 102006023186
(43) Veröffentlichungstag der Anmeldung: 25.02.2009
(73) Patentinhaber: Fraunhofer-Gesellschaft zur Förderung der angewandten Forschung e.V., 80686 München (DE)
(72) Erfinder: SCHMITT, Katrin, 79108 Freiburg (DE); HOFFMANN, Christian, 79115 Freiburg (DE)
(74) Vertreter: Grünecker, Kinkeldey, Stockmair & Schwanhäusser
(86) Internationale Anmeldenummer: PCT/EP2007/003125
(87) Internationale Veröffentlichungsnummer: WO 2007/131577

(56) Entgegenhaltungen:
- EP-A- 1 281 969
- WO-A-01/62170
- WO-A-97/04836
- US-A- 5 190 761

## Beschreibung

Die vorliegende Erfindung betrifft ein transdermales therapeutisches System, das insbesondere zur Therapie direkt am Patienten, beispielsweise von chronischen Wunden, oder in der Chemotherapie verwendbar ist.

Transdermale Systeme sind eine Form der Medikation eines Patienten, bei welcher ein Wirkstoff, meist auf einem Pflaster, über eine gewisse Zeit hinweg (häufig mehrere Stunden oder Tage) über die Haut des Patienten in die darunterliegenden Gefäße oder das darunterliegende Gewebe aufgenommen wird.

Diese Form der Medikation ermöglicht eine Umgehung des First-Pass Metabolismus, also der ersten Leberpassage. Hierdurch kann eine notwendige Dosierung des Wirkstoffes vermindert werden. Somit können organ- und kreislaufbelastende Überdosierungen vermieden werden.

Ein bekanntes transdermales therapeutisches System sind "passive" Pflaster, wie etwa Fentanylpflaster zur Tumorschmerztherapie oder Nikotinpflaster zur Entwöhnung bei Rauchern. Diese "passiven" Pflaster werden über einen gewissen Zeitraum von beispielsweise bis zu 72 Stunden auf der Haut belassen und geben kontinuierlich ihren Wirkstoff aus einer Klebeschicht ab. Anschließend wird das Pflaster ausgetauscht.

Hierdurch kann der Wirkstoff zwar perkutan über einen gewissen Zeitraum kontinuierlich abgegeben werden, allerdings ist dieser Abgabevorgang während der Applikation auf der Haut nicht aktiv steuerbar.

Es ist jedoch vorteilhaft, der Wirkstoff dosiert über einen bestimmten Zeitraum abzugeben, da dann eine individuelle Medikation des Patienten ermöglicht ist. Ein steuerbares transdermales therapeutisches System ermöglicht also eine Gesamtmenge an abgegebenem Wirkstoff bei gleicher therapeutischer Effizienz zu minimieren.

Ein derartiges steuerbares System sind elektrochemisch arbeitende transdermale therapeutische Systeme, in welchen der Wirkstoff in einer Klebeschicht gespeichert ist. Mittels zweier Elektroden wird ein elektrisches Feld an die Klebeschicht angelegt. Aufgrund der Wirkung des angelegten elektrischen Feldes wird der Wirkstoff dosiert abgegeben.

Bei dieser elektrochemisch arbeitenden Abgabemethode muß jedoch das jeweilige Wirkstoffmolekül eine entsprechende Ladung tragen. Zudem ist die abgegebene Dosis über eine Höhe der Potentialdifferenz und einer Dauer des angelegten elektrischen Feldes vorgegeben.

Außerdem beruhen alle bislang verfügbaren transdermalen therapeutischen Systeme auf einer Immobilisierung eines Wirkstoffes in einer Klebeschicht, welche bei den betreffenden Patienten vielfach Kontaktallergien auslöst, und dies unabhängig vom Wirkstoff selbst.

Es ist daher eine Aufgabe der vorliegenden Erfindung, ein verbessertes transdermales therapeutisches System mit einer steuerbaren Wirkstoffdosierung anzugeben.

Erfindungsgemäß wird die vorliegende Aufgabe gelöst durch ein transdermales therapeutisches System mit den Merkmalen des Anspruchs 1. Bevorzugte Ausführungsformen sind Gegenstand der abhängigen Ansprüche.

Mit dem erfindungsgemäßen transdermalen therapeutischen System ist eine optisch steuerbare Wirkstoffabgabe und -dosierung ermöglicht.

Beim erfindungsgemäßen transdermalen therapeutischen System ist der abzugebende Wirkstoff an einer Oberfläche des Wellenleiters vermittels einer spaltbaren Verbindung immobilisiert Aufgrund eines elektromagnetischen Impulses im Wellenleiter und einer entsprechenden Spaltung der Halteverbindung erfolgt eine kontrollierte Wirkstoffabgabe.

Hierdurch ist eine zeitliche Applikation des Wirkstoffes (Dosierung) ermöglicht Entsprechend kann eine therapeutische Effizienz gesteigert werden. Zudem ist eine Kostenreduktion ermöglicht, insbesondere wenn nur geringe Mengen des Wirkstoffes über einen längeren Zeitraum eingesetzt werden stollen. Das erfindungsgemäße transdermale therapeutische System ermöglicht zudem eine breite Anwendung von Wirkstoffmolekülen, da sowohl geladene als auch ungeladene Wirkstoffmoleküle an der Oberfläche des Welienleiters immobilisiert werden können. Bei dem erfindungsgemäßen transdermalen therapeutischen System kann auch auf eine Klebeschicht verzichtet werden, wodurch Kontaktallergien gegen diese Klebeschicht ausgeschlossen sind.

Vorzugsweise ist der Wellenleiter als zumindest eine Polymerfaser, insbesondere als ein Polymerfaserverbund, ausgebildet. Alternativ hierzu kann der Wellenleiter als Polymerschicht, insbesondere als planare Trägerfolie ausgebildet sein, wobei hierbei die Polymerschicht vorzugsweise luftdurchlässig ist.

Weiterhin kann der Wellenleiter als Lichtwellenleiter ausgebildet sein, wobei bevorzugterweise Licht im sichtbaren oder ultravioletten Wellenlängenbereich oder IR-Licht verwendbar ist. Bei Verwendung von UV-Licht ist vorteilhaft, daß das transdermale therapeutische System (insbesondere bei Ausbildung als Wundverband oder Pflaster oder Wundauflage) zeitweise auch sichtbarem Licht ausgesetzt werden kann. Das UV-Licht ermöglicht zudem eine gleichzeitige desinfizierende Wirkung auf eine Hautoberfläche, was bei längerem Verbleib des Pflasters/Wundverbandes auf der Haut einen weiteren Vorteil bietet.

Erfindungsgemäß ist die spaltbare Verbindung ein photolabiler Linker, welcher durch eine Wirkung eines durch Lichtimpulse im Lichtleiter erzeugten Evaneszentfeldes spaltbar ist. Dabei kann eine Lichtintensität im Evaneszentfeld so gering gehalten werden, daß auch bei Verwendung von UV-Licht trotz Desinfizierung der Hautoberfläche eine Schädigung der Haut in jedem Falle vermeidbar ist

Das transdermale therapeutische System kann ebenso ein optisch basiertes Steuerungssystem zur Steuerung der Erzeugung der elektromagnetischen Impulse zur dosierten Freigabe des Wirkstoffes aufweisen. Insbesondere kann eine Lichtquelle zur Erzeugung der Lichtimpulse, insbesondere eine Leuchtdiode im UV-Bereich, verwendet werden.

Weiterhin kann eine Optik zur Einkoppelung der elektromagnetischen Impulse in den Wellenleiter vorgesehen sein.

Bevorzugterweise ist das transdermale therapeutische System außerdem in Segmente unterteilt, wobei die einzelnen Segmente zur dosierten Freigabe des Wirkstoffes sequentiell ansteuerbar sind.

Gemäß einem weiteren bevorzugten Ausführungsbeispiel ist ein Sensor zur Ermittlung einer Dosis an freigegebenem Wirkstoff vorgesehen. Dieser Sensor kann eine Diode oder ein Diodenarray zur Messung einer Extinktion in Abhängigkeit der Oberflächenbelegungsdichte zur Ermittlung der Dosis an freigegebenem Wirkstoff umfassen.

Das transdermale therapeutische System kann ferner eine Trägerschicht aufweisen, auf welcher der Wellenleiter angeordnet ist. Diese Trägerschicht kann hierbei als Pflaster, Wundauflage oder Wundverband ausgebildet sein.

Die vorliegende Erfindung wird nachfolgend anhand von bevorzugten Ausführungsbeispielen in Verbindung mit den zugehörigen Zeichnungen näher erläutert. In diesen zeigen:
- Fig. 1: ein Ausführungsbeispiel des transdermalen therapeutischen Systems zur optisch gesteuerten Wirkstoffdosierung,
- Fig. 2A: ein Ausführungsbeispiel eines photolabilen Linkers im UV-Bereich,
- Fig. 2B: ein weiteres Ausführungsbeispiel eines photolabilen Linkers im UV-Bereich, und
- Fig. 3: ein zeitlicher Verlauf der Wirkstoffdosierung bei verschiedenen Verabreichungsformen, einschließlich der optisch gesteuerten Wirkstoffdosierung beim vorliegenden transdermalen therapeutischen System.

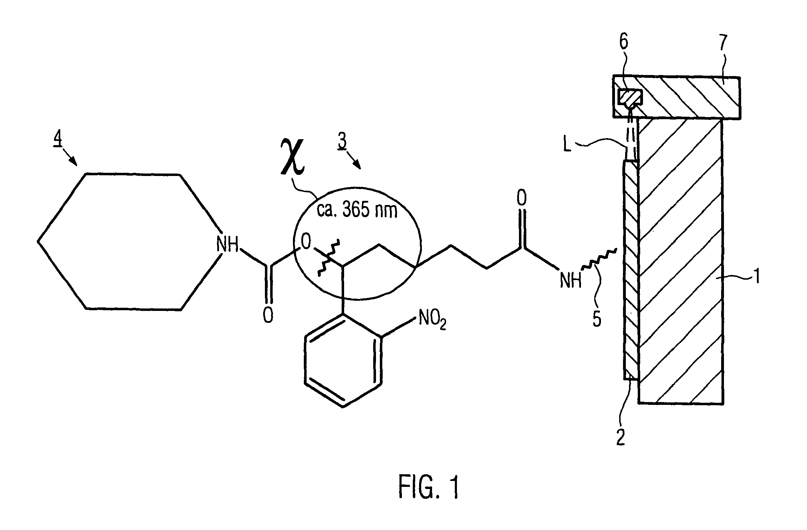

Das in Fig. 1 gezeigte transdermale therapeutische System weist ein Trägermaterial 1 auf, wobei das Trägermaterial als Pflaster, Verband, Wundauflage oder ähnliches ausgebildet ist. Hierdurch wird eine Applikation auf der Haut erleichtert.

Auf dem Trägermaterial 1 ist eine (partielle) Polymerbeschichtung 2 als Wellenleiter angeordnet. Das vorliegende transdermale therapeutische System, also das Pflaster oder der Wundverband oder die Wundauflage, ist also mit einer (idealerweise luftdurchlässigen) Polymerschicht bzw. mit einem Netz von Polymerfasern versehen, die als Lichtwellenleiter 2 ausgebildet sind.

Auf diesem Lichtwellenleiter 2, d.h. auf der Polymeroberfläche, sind photolabile Linker 3 immobilisiert, an welchen wiederum der Wirkstoff 4 gebunden ist.

Der Photolinker besteht aus zwei funktionellen Gruppen, die dazu dienen, die Verbindung zum Wirkstoff und die Verbindung zur Wellenleiterobefläche herzustellen. Eine mögliche Immobilisierungsstrategie ist das Modifizieren der Oberfläche, z.B. nasschemisch oder durch Plasmabehandlung, um funktionelle Gruppen an dieser zu erzeugen. Dafür eignen sich beispielsweise (primäre) Amino- oder Hydroxygruppen. Ist das Linkermolekül mit einem (Aktiv)ester versehen (z.B. N-hydrosuccinimidester) oder wird dieser beispielsweise mit EDC aktiviert, so kann dieser mit den Amino- oder Hydroxygruppen an der Wellenleiteroberfläche zum Carbonsäureamid beziehungsweise zum Ester reagieren. Somit ist das Linkermolekül kovalent an der Oberfläche verankert. Ebenso muss das Wirkstoffmolekül mit dem Linker versehen werden. Dafür können ähnliche chemische Reaktionen durchgeführt werden wie an der Wellenleiteroberfläche. Gegebenfalls muss über eine Schutzgruppenstrategie eine Ringbildung an der Oberfläche beziehungsweise eine Polymerisation verhindert werden. Neben den beiden funktionellen Gruppen für die Reaktion mit dem Wirkstoffmolekül sowie mit der Oberfläche muss das Molekül eine photolabile Bindung aufweisen, um den Wirkstoff freizusetzen. Bei der Reaktion zwischen Wirkstoffmolekül und Photolinker ist darauf zu achten, dass bei Freisetzung ein möglicher Rest des Photolinkers am Wirkstoffmolekül sich nicht nachteilig in der Wirkung (Wirkung und Nebenwirkung) verhält.

Der Aufbau des photolabilen Linkers 3 ist in Fig. 1 nur beispielhaft angegeben, wobei die Immobilisierung des photolabilen Linkers 3 an der Polymeroberfläche über eine gewellte Linie 5 symbolisch angedeutet ist.

Auch der Wirkstoff (das Medikament) 4 ist lediglich symbolisch über ein Hexagon angedeutet, ohne daß hierdurch eine bestimmte Struktur des Wirkstoffes vorgegeben werden soll.

Der photolabile Linker 3 kann durch Lichtimpulse L im Wellenleiter 2 gespalten werden. Dieser Vorgang ist im mit X bezeichneten Kreis für eine beispielhafte Wellenlänge im UV-Lichtbereich von λ= 365 nm über die weitere Wellenlinie angedeutet. Somit setzt das transdermale therapeutische System bei Spaltung des photolabilen Linkers 3 den Wirkstoff 4 frei.

Der Lichtimpuls L wird mittels der Lichtquelle 6 erzeugt und über eine Optik in den Lichtwellenleiter 2 eingekoppelt.

Die Lichtquelle 6 wird über die Elektronik 7 gesteuert.

Es ist jedoch auch denkbar in einem weiteren Ausführungsbeispiel den Lichtwellenleiter zumindest abschnittsweise als photonischer Kristall auszubilden, wobei die Lichtquelle dann in den Bereich des photonischen Kristalls integriert werden kann. Der photonische Kristall ermöglicht ein Filtern der beispielsweise durch einen thermischen Strahlen abgegebenen breitbandigen Strahlung. Eine separate Optik zum Einkoppeln der Strahlung ist bei diesem Ausführungsbeispiel nicht notwendig.

Ebenfalls ist möglich, in einem Teilbereich des photonischen Kristalls ein elektrisch leitendes und/oder magnetisches Metall vorzusehen, welches durch die Wirkung eines elektrischen und/oder magnetischen Feldes erhitzt werden kann. Auf diese Weise kann die Lichtquelle integral im Wellenleiter ausgebildet werden. Zudem entfällt dann auch das Einkoppeln des Lichtes in den Wellenleiter vollständig.

Im Wellenleiter 2 eingekoppelte (oder in diesem wie vorstehend erläutert erzeugte) Lichtwellen L erzeugen im Umfeld des Lichtwellenleiters 2 ein Evaneszentfeld. Der photolabile Linker 3 wird durch die Wirkung des durch Lichtimpulse im Lichtwellenleiter erzeugten Evaneszentfeldes gespalten und setzt somit den Wirkstoff frei, was einen erheblichen Vorteil gegenüber einer lediglich kontinuierlichen Freigabe darstellt. Photolabile Linker (auch photosensitive Linker genannt) gibt es für einen großen Wellenlängenbereich, sowohl im sichtbaren Licht als auch im UV-Lichtbereich. Eine mögliche Lichtquelle 6 für die Anwendung des transdermalen therapeutischen Systems wären daher kommerziell erhältliche UV-LED's, z.B. im Bereich von λ= 365 nm, wie in Fig. 1 angedeutet.

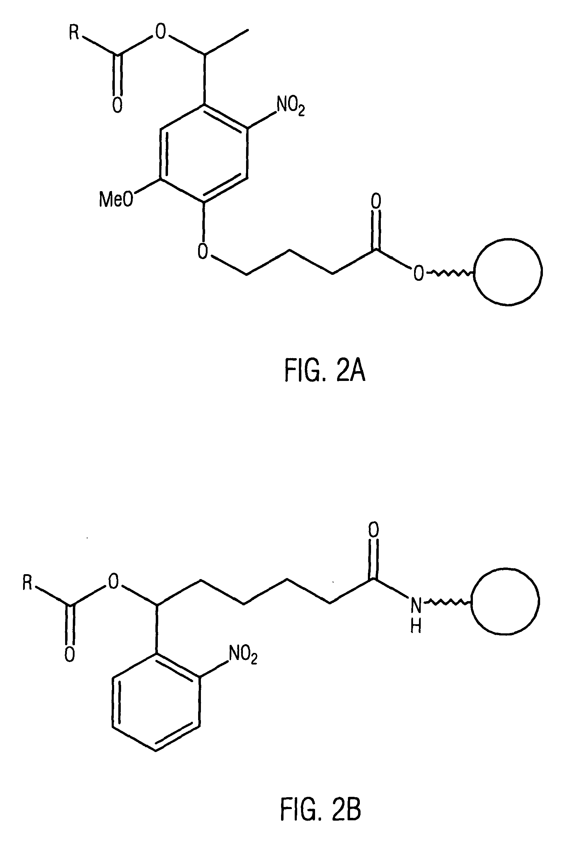

In diesem Wellenlängenbereich existieren geeignete photolabile Linker, von denen zwei in den Fig. 2A und 2B beispielhaft dargestellt sind. Eine Aufstellung verschiedener bei der vorliegenden Erfindung verwendbarer photosensitiver Linker findet sich in Bannwarth, W., Hinzen, B., Combinatorial Chemistry, Vol. 26, Wiley-VCH.

Das hier beschriebene transdermale therapeutische System beruht also auf der Spaltung der photolabilen Linker im Evaneszentfeld des Lichtwellenleiters, was eine Verwendung von UV-Licht möglich macht, ohne daß die Haut des Patienten geschädigt wird, da die Lichtleistung im Evaneszentfeld gering gehalten werden kann. Bei Anwendung von UV-Licht kann zudem auf eine Abschirmung des photolabilen Linkers gegen Tageslicht verzichtet werden. Da Wundverbände oder auch Pflaster normalerweise zumindest zeitweise sichtbarem Licht ausgesetzt sind, wäre bei Verwendung von Wellenlängenbereichen des sichtbaren Lichtes eine solche Abschirmung erforderlich. Folglich ermöglicht die Verwendung von UV-Licht eine unproblematische Applikation des transdermalen therapeutischen Systems auch bei Tageslicht. Gleichzeitig verhindert die Verwendung des UV-Lichtes durch seine antimikrobielle Wirkung eine Ausbreitung von Keimen oder ähnlichem an der Polymeroberfläche, wodurch ein Risiko von Allergien oder Infektionen zusätzlich vermindert wird.

Im Gegensatz zu dem eingangs beschriebenen elektrochemisch basierten steuerbaren transdermalen therapeutischen System, können bei dem hier beschriebenen transdermalen therapeutischen System mit optisch gesteuerter Wirkstoffdosierung auch ungeladene Moleküle zur Anwendung kommen, wodurch ein Anwendungsspektrum dieses transdermalen therapeutischen Systems erheblich vergrößert ist.

Da bei einer direkten Immobilisierung des Wirkstoffes über neutrale photolabile Linker eine Klebeschicht zwischen Trägermaterial und Wirkstoff entfällt, wird das Risiko einer Kontaktallergie beim Patienten minimiert.

Ein optisch basiertes Steuerungssystem beinhaltet zudem die Möglichkeit, über entsprechend Sensorik die tatsächliche Dosis an freigegebenem Wirkstoff festzustellen und ggf. nach Übertragung der Daten an den behandelnden Arzt direkt in die weitere Therapie einzugreifen. Eine Möglichkeit der Erfassung der tatsächlich abgegebenen Wirkstoffdosis durch Integration entsprechender Sensoren ist die Messung der Extinktion des Lichtes im Lichtwellenleiter in Abhängigkeit der Oberflächenbelegungsdichte mittels eines Diodenarrays.

Mögliche Anwendungsfelder des vorliegenden transdermalen therapeutischen Systems sind die Therapie von chronischen Wunden oder die Chemotherapie.

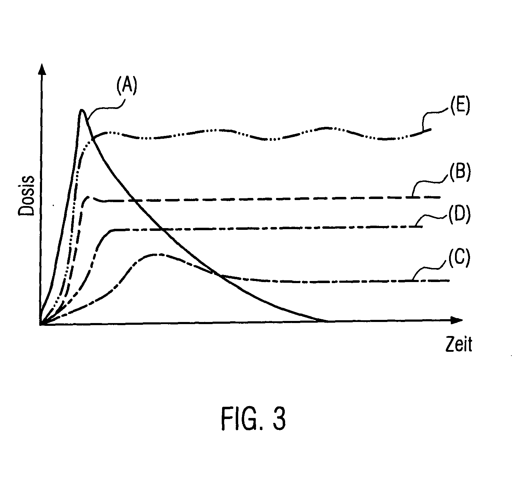

Ein Vergleich des zeitlichen Verlaufs der Wirkstoffabgabe bei verschiedenen Verabreichungsformen ist in Fig. 3 dargestellt. In Fig. 3 ist die entsprechende Dosis an Wirkstoff über dem Zeitverlauf aufgetragen.

Dabei zeigt die Kurve A (durchgezogene Linie) den Wirkstoffverlauf für eine Verabreichung mittels Injektion oder Tablette. Der rasche Anstieg der Wirkstoffmenge sowie der anschließende ebenfalls rasche Abfall der Wirkstoffdosis ist deutlich erkennbar.

In Kurve B (gestrichelte Linie) ist der zeitliche Verlauf der Wirkstoffdosis für ein passives transdermales therapeutisches System dargestellt, wobei der rasche Anstieg in Verbindung mit der zeitlich konstanten Wirkstoffabgabe deutlich wird.

Die Kurven C, D und E (strich-punktierte Linien) stellen individuelle Wirkstoffdosisabgaben mit dem vorliegenden transdermalen therapeutischen System dar. Die Anpaßbarkeit der Wirkstoffdosisabgabemenge pro Zeit wird hierdurch deutlich.

Bei den vorliegenden Ausführungsbeispielen ist eine Immobilisierung von photolabilen Linkern über ein breites Wellenlängenspektrum möglich, wobei ein besonderer Vorteil der Verwendung einer UV-Lichtquelle die unproblematische Applikation des transdermalen therapeutischen Systems bei Tageslicht ist. Weiterhin ist auch die Ausnutzung der antimikrobiellen Wirkung des UV-Lichtes möglich.

Als "Substrat" für den photolabilen Linker kommt neben einer Polymerfaser bzw. einem Geflecht/Netz aus Polymerfasern auch eine planare Trägerfolie in Betracht, in die Licht entsprechend eingekoppelt wird.

Daneben können als Substrat auch organische, halbleitende Polymere verwendet werden, die zur Herstellung von OLED's (organische Leuchtdioden) eingesetzt werden. Hierbei ist insbesondere ein Aufbau, bestehend aus mehreren organischen Schichten, möglich.

Ein Verbund von Polymerfasern ist insoweit vorteilhaft, als auf Grund der vergrößerten Oberfläche eine höhere Wirkstoffkapazität mit sich bringt.

Zwei mögliche photolabile Linker sind in den Fig. 2A und 2B dargestellt. Prinzipiell stehen aber eine Vielzahl an möglichen photolabilen Linkem zur Verfügung, auch für andere als die vorliegend spezifizierte Wellenlänge.

## Patentansprüche

1. Transdermales therapeutisches System zur steuerbaren Abgabe eines Wirkstoffs mit einem Wellenleiter, wobei der Wirkstoff über einen fotolabilen Linker, der durch eine Wirkung eines durch Lichtimpulse im Wellenleiter erzeugten Evaneszentfeldes spaltbar ist, am Wellenleiter angelagert ist.

2. Transdermales therapeutisches System nach Anspruch 1, **dadurch gekennzeichnet, dass** der Wellenleiter als zumindest eine Polymerfaser oder als ein Polymerfaserverbund ausgebildet ist.

3. Transdermales therapeutisches System nach Anspruch 1, **dadurch gekennzeichnet, dass** der Wellenleiter als Polymerschicht, insbesondere als plane Trägerfolie, ausgebildet ist.

4. Transdermales therapeutisches System nach Anspruch 3, **dadurch gekennzeichnet, dass** die Polymerschicht luftdurchlässig ist.

5. Transdermales therapeutisches System nach einem der Ansprüche 1 bis 4, **dadurch gekennzeichnet, dass** der Wellenleiter als Lichtwellenleiter ausgebildet ist.

6. Transdermales therapeutisches System nach Anspruch 5, **gekennzeichnet durch** eine Verwendung von Licht im sichtbaren oder ultravioletten Wellenlängenbereich oder eine Verwendung von Infrarotem Licht.

7. Transdermales therapeutisches System nach einem der Ansprüche 1 bis 6, **gekennzeichnet durch** ein optisch basiertes Steuerungssystem zur Steuerung der Erzeugung der elektromagnetischen Impulse zur dosierten Freigabe des Wirkstoffes.

8. Transdermales therapeutisches System nach Anspruch 7, **gekennzeichnet durch** eine Lichtquelle zur Erzeugung der Lichtimpulse, insbesondere eine Leuchtdiode im UV-Bereich.

9. Transdermales therapeutisches System nach einem der Ansprüche 1 bis 8, **gekennzeichnet durch** eine Optik zur Einkopplung der elektromagnetischen Impulse in den Wellenleiter.

10. Transdermales therapeutisches System nach einem der Ansprüche 1 bis 9, **dadurch gekennzeichnet, dass** das transdermale therapeutische System in Segmente unterteilt ist, wobei die einzelnen Segmente zur dosierten Freigabe des Wirkstoffes sequentiell ansteuerbar sind.

11. Transdermales therapeutisches System nach einem der Ansprüche 1 bis 10, **gekennzeichnet durch** einen Sensor zur Ermittlung einer Dosis an freigegebenem Wirkstoff.

12. Transdermales therapeutisches System nach Anspruch 11, **gekennzeichnet durch** eine Diode oder ein Diodenarray zur Messung einer Extinktion in Abhängigkeit der Oberflächenbelegungsdichte zur Ermittlung der Dosis an freigegebenem Wirkstoff.

13. Transdermales therapeutisches System nach einem der Ansprüche 1 bis 12, **gekennzeichnet durch** eine Trägerschicht, auf welcher der Wellenleiter angeordnet ist.

14. Transdermales therapeutisches System nach Anspruch 13, **dadurch gekennzeichnet, dass** die Trägerschicht als Pflaster, Wundauflage oder Wundverband ausgebildet ist.

## Claims

1. A transdermal therapeutic system for controllably releasing an active agent, said system comprising a waveguide, wherein the active agent is attached to the waveguide via a photolabile link which is cleavable by the action of an evanescent field produced by light pulses in the waveguide.

2. The transdermal therapeutic system according to claim 1, **characterized in that** the waveguide is formed as at least one polymer fiber or as a compound of polymer fibers.

3. The transdermal therapeutic system according to claim 1, **characterized in that** the waveguide is formed as a polymer layer, particularly as a planar carrier film.

4. The transdermal therapeutic system according to claim 3, **characterized in that** the polymer layer is air-permeable.

5. The transdermal therapeutic system according to any of claims 1 to 4, **characterized in that** the waveguide is formed as an optical waveguide.

6. The transdermal therapeutic system according to claim 5, **characterized by** a use of light in the visible or ultraviolet wavelength range or by a use of infrared light.

7. The transdermal therapeutic system according to any of claims 1 to 6, **characterized by** an optically based control system for controlling the generating of the electromagnetic pulses for a dosed release of the active agent.

8. The transdermal therapeutic system according to claim 7, **characterized by** a light source for generating the light pulses, particularly by a light emitting diode in the UV range.

9. The transdermal therapeutic system according to any of claims 1 to 8, **characterized by** an optical system for coupling the electromagnetic pulses into the waveguide.

10. The transdermal therapeutic system according to any of claims 1 to 9, **characterized in that** the transdermal therapeutic system is sub-divided into segments, the individual segments being sequentially activatable for a dosed release of the active agent.

11. The transdermal therapeutic system according to any of claims 1 to 10, **characterized by** a sensor for determining a dose of released active agent.

12. The transdermal therapeutic system according to claim 11, **characterized by** a diode or a diode array for measuring an extinction in dependence of the surface covering density for determining the dose of released active agent.

13. The transdermal therapeutic system according to any of claims 1 to 12, **characterized by** a carrier layer on which the waveguide is arranged.

14. The transdermal therapeutic system according to claim 13, **characterized in that** the carrier layer is formed as a patch, a wound dressing or a wound bandage.

## Revendications

1. Système thérapeutique transdermique destiné à la libération contrôlée d'un principe actif, comprenant un guide d'ondes, système dans lequel le principe actif est fixé sur le guide d'ondes par l'intermédiaire d'une liaison photo-labile, qui peut être dissociée sous l'effet d'un champ d'évanescence produit par des impulsions lumineuses dans le guide d'ondes.

2. Système thérapeutique transdermique selon la revendication 1, **caractérisé en ce que** le guide d'ondes est réalisé sous la forme d'au moins une fibre polymère ou d'un composite de fibres polymères.

3. Système thérapeutique transdermique selon la revendication 1, **caractérisé en ce que** le guide d'ondes est réalisé sous forme de couche de polymère, notamment sous forme de feuille de support plane.

4. Système thérapeutique transdermique selon la revendication 4, **caractérisé en ce que** la couche de polymère est perméable à l'air.

5. Système thérapeutique transdermique selon l'une des revendications 1 à 4, **caractérisé en ce que** le guide d'ondes est réalisé sous forme de guide d'ondes lumineuses.

6. Système thérapeutique transdermique selon la revendication 5, **caractérisé par** une utilisation de lumière dans le domaine des longueurs d'ondes visibles ou de l'ultraviolet, ou une utilisation de lumière infrarouge.

7. Système thérapeutique transdermique selon l'une des revendications 1 à 6, **caractérisé par** un système de commande basé sur un principe optique pour commander la production des impulsions électromagnétiques, en vue de la libération dosée du principe actif.

8. Système thérapeutique transdermique selon la revendication 7, **caractérisé par** une source de lumière pour produire les impulsions de lumière, notamment une diode luminescente dans le domaine UV.

9. Système thérapeutique transdermique selon l'une des revendications 1 à 8, **caractérisé par** une optique pour l'injection ou le couplage des impulsions électromagnétiques dans le guide d'ondes.

10. Système thérapeutique transdermique selon l'une des revendications 1 à 9, **caractérisé en ce que** le système thérapeutique transdermique est subdivisé en segments, les segments individuels pouvant être commandés séquentiellement pour la libération dosée du principe actif.

11. Système thérapeutique transdermique selon l'une des revendications 1 à 10, **caractérisé par** un capteur pour déterminer une dose de principe actif libéré.

12. Système thérapeutique transdermique selon la revendication 11, **caractérisé par** une diode ou un réseau de diodes pour mesurer une extinction fonction de la densité d'occupation de surface, en vue de déterminer la dose de principe actif libéré.

13. Système thérapeutique transdermique selon l'une des revendications 1 à 12, **caractérisé par** une couche de support sur laquelle est agencé le guide d'ondes.

14. Système thérapeutique transdermique selon la revendication 13, **caractérisé en ce que** la couche de support est réalisée sous forme de sparadrap ou timbre, de pansement de plaie ou de bandage de plaie.
